# EUROPEAN PATENT SPECIFICATION

(11) **EP 2 406 367 B1**
(45) Date of publication and mention of the grant of the patent: **20.05.2015**
(21) Application number: 10751267.5
(22) Date of filing: 09.03.2010
(51) Int. Cl.: C12M 1/00

(54) **SEPARATION OF ANTIGEN-SPECIFIC MEMORY B CELLS WITH A CONJUGATED BIOPOLYMER SURFACE**
TRENNUNG VON ANTIGENSPEZIFISCHEN GEDÄCHTNIS-B-ZELLEN MIT KONJUGIERTER BIOPOLYMERFLÄCHE
SÉPARATION DE LYMPHOCYTES B MÉMOIRE SPÉCIFIQUES D'ANTIGÈNES À L'AIDE D'UNE SURFACE DE BIOPOLYMÈRE CONJUGUÉ À UN LIGAND

(30) Priority: 09.03.2009 US 158649 P
(43) Date of publication of application: 18.01.2012
(73) Proprietor: Biofactura, Inc., Rockville, MD 20850 (US)
(72) Inventor: SAMPEY, Darryl, B., Frederick MD 21701 (US)
(74) Representative: Potter Clarkson LLP
(86) International application number: PCT/US2010/026624
(87) International publication number: WO 2010/104828

(56) References cited:
- WO-A1-99/58977
- WO-A1-2008/118926
- WO-A1-2008/130655
- DEBANJAN SARKAR ET AL.: 'Chemical enginnering of mesenchymal stem cells to induce a cell rolling response.' BIOCONJUGATE CHEM. vol. 19, 2008, pages 2105 - 2109, XP055100140
- ROHIT KARNIK ET AL.: 'Nanomechanical control of cell rolling in two dimensions through surface patterning of receptors.' NANO LETTERS. vol. 8, no. 4, 2008, pages 1153 - 1158, XP008123376
- MICHAEL R. KING ET AL.: 'Multiparticle adhesive dynamics: hydrodynamic recruitment of rolling leukocytes.' PNAS. vol. 98, no. 26, 2001, pages 14919 - 14924, XP055100326

## Description

### BACKGROUND

The potential use of viruses (e.g., poxviruses, filoviruses) as bioweapons is a growing concern, especially with regard to human diseases for which effective countermeasures are presently unavailable. Therapeutic antibodies represent a relevant strategy for treatment and/or prophylaxis of individuals exposed to or infected by viral disease agents of significance to the military and anti-bioterrorism efforts. Currently, vaccinia virus (VACV) immune globulin (VIG), a human blood-derived polyclonal Ig product, is the "first line" therapy for treatment of disseminated VACV infection stemming from adverse smallpox vaccination events. In addition, a cross-reactive mAb formulation could serve as a therapeutic or prophylactic for infections caused by other pathogenic orthopoxviruses such as monkeypox virus and variola virus, the causative agent of smallpox, thereby mitigating the threat of bioterrorism with such viral agents.

Of the approximately 200 genes contained in the VACV genome, only a few encode proteins that are known to induce a neutralizing antibody response, including H3, A17, A27, D8, L1, and B5¹. A seventh gene, A33R, encodes a protein that elicits a non-neutralizing antibody response but is, nevertheless, protective. Based historic and preliminary data, it is likely that combinations of VACV antigen-specific mAbs that exhibit protective efficacy in animal models will be effective for treatment of disease in humans. One method used to identify Fabs to A33R, B5R, and L1R is the pCOMB3 phage display system (Scripps Institute). This method can be problematic, for example, due to low library size/diversity, inefficient cloning, and stability problems associated with the first-generation pCOMB3 system.

With the approval of Humira in 2003, the industry has seen a full shift of new antibody therapeutics from chimeric to humanized to fully-human sequences. The objective in the industry is to manufacture a drug that is identical to that which is produced in the human body. Currently available methods suffer from various problems, for example, the high cost of discovering and manufacturing antibody-based drugs and difficulties with speed, stability, fidelity, regulatory compliance, and product expression.

Immunotherapeutics such as monoclonal antibodies have been proven to be lifesaving medical products and key reagents for diagnostic and research tools. There remains a need in the art for improved methods and devices for the identification and production of fully humanized monoclonal antibodies having a desired specificity. The present invention provides a device for the isolation of antigen- specific memory B cells. These cells are uniquely designed to express and secrete antibodies. The genetic, biochemical, and cellular organelle composition of cell lines derived from human B cells will accurately and faithfully produce the most human therapeutic molecule possible. This device may be used for the discovery and manufacture of new generation immunotherapeutics to meet critical needs in areas such as infectious disease, oncology, public health, and biodefense.

WO 2008/130,655 discloses methods of quantifying cells in a sample by lysing the cells and measuring at least one intracellular component.

### BRIEF SUMMARY OF THE INVENTION

A first aspect of the invention provides (as set out in claim 1) a device for the separation of immune cells by interactions of receptors of said immune cells with ligands wherein the ligands are immobilized in a concentration gradient on a functionalized surface of the device and the device is adapted to flow the immune cells across the functionalized surface, wherein the concentration gradient of the ligands increases along a direction of flow of the immune cells across the functionalized surface.

The invention further provides the device of claim 1, wherein said device is a flow cell or microfluidics device.

The invention further provides the device of claim 1, wherein said immune Cells are B cells.

The invention further provides the device of claim 1, wherein said immune cell receptors are B cell receptors.

The invention further provides the device of claim 1, wherein said ligands are antigens with B cell epitopes.

The invention further provides the device of claim 1, wherein said immune cells are T cells.

The invention further provides the device of claim 1, wherein said immune cell receptors are T cell receptors.

The invention further provides the device of claim 1, wherein said ligands are antigens with T cell epitopes.

The invention further provides the device of claim 1, wherein said functionalized surface is a polymer conjugated to said ligands.

The invention further provides a method for isolating immune cells, comprising: contacting a solution comprising the immune cells with a device according to claim 1.

The invention further provides the method according to claim 10, wherein the immune cells are arrested on said functionalized surface and are caused to proliferate by induction of activation.

The invention further provides the method according to claim 11, wherein viable activated proliferating immune cell colonies are recovered from the device.

The invention further provides the method according to claim 12, wherein colonies are recovered by releasing the ligand from the polymer.

The invention further provides the method according to claim 12, wherein the colonies are mechanically removed.

In one embodiment, the present invention provides a device for the separation of immune cells by interactions of receptors of said immune cells with ligands as defined by the claims. Typically, the ligands are immobilized on a functionalized surface of the device and the device is adapted to flow the immune cells across the functionalized surface. Any type of ligand may be used, for example, ligands bound by receptors present on the immune cells. Suitable examples include, but are not limited to, antigens for which the immune cells are specific. In some embodiments, devices of the invention may be flow cells and/or microfluidics device.

Devices of the invention, as defined by the claims, may be used to isolate any type of immune cell, for example, neutrophils, eosinophils, basophils, lymphocytes, and/or monocytes. In some embodiments, cells to be isolated using the device of the invention may be lymphocytes, for example, B cells, T-cells, and/or natural killer cells. In some embodiments, cell to be isolated may be B cells.

Any receptor expressed on the surface of an immune cell may be used to isolate the cell by attaching one or more ligands bound by the receptor on the functionalized surface of the device as defined by the claims. Suitable receptors include B cell receptors for which suitable ligands include, but are not limited to antigens with B cell epitopes. In another embodiment, the immune cell receptors may be T cell receptors for which suitable ligands include, but are not limited to, antigens with T cell epitopes.

Any suitable method known in the art may be used to functionalize the surface of the device, as defined by the claims, upon which immune cells are to be isolated. In some embodiments, the surface may be functionalized by depositing a polymer on the surface. One or more ligands may be conjugated to the polymer before or after deposition.

The present invention provides methods of isolating immune cells by contacting a solution comprising the immune cells with a device of the invention as defined by the claims. In some embodiments, methods of the invention may comprise arresting (i.e., immobilizing) immune cells the functionalized surface. Such immune cells may be caused to proliferate by induction of activation. Such activation may result in the formation of colonies of immune cells. In some methods, viable activated proliferating immune cell colonies are recovered from the device. Colonies may be recovered using any method known in the art, for example, colonies may be recovered by releasing the ligand from the polymer and/or colonies may be mechanically (e.g., pipetted) removed.

In some embodiments, the present invention, as defined by the claims, may be used to separate viral antigen-specific memory B cells from human peripheral blood mononuclear cells (PBMCs) derived from a vaccinated donor by indirect affinity magnetic bead methods. Materials and methods of the invention, as defined by the claims, may include antigen conjugated biopolymer surfaces that can be used to mediate antigen specific B cell separation by differential rolling adhesion. In some embodiments, the present invention, as defined by the claims, provides antigen conjugated biopolymer surfaces for antigen specific B cell separation. Experiments with antibody-conjugated microspheres will be used to evaluate surface gradients, shear rates, and flow cell design prior to experiments with B cells.

In some embodiments, B cells isolated using the present invention, as defined by the claims, may be activated *in situ* for clone isolation. For example, biopolymer surface-captured antigen-specific B cells can be activated *in situ* for clone isolation.

In some embodiments, the present invention, as defined by the claims, may be used to isolate antigen-specific B cells which can then be activated, induced to differentiate to antibody secreting cells (ASCs), and induced to expand and form colonies on the biopolymer surface. Colonies of activated antigen-specific memory B cells can be isolated from a biopolymer surface to generate IgG-secreting cell lines. Thus, the present invention provides materials and methods that may be used to isolate, expand, and characterize IgG-expressing cell lines derived from activated antigen-specific B cell colonies and generate clonal antibody-secreting cell lines.

### BRIEF DESCRIPTION OF THE DRAWINGS

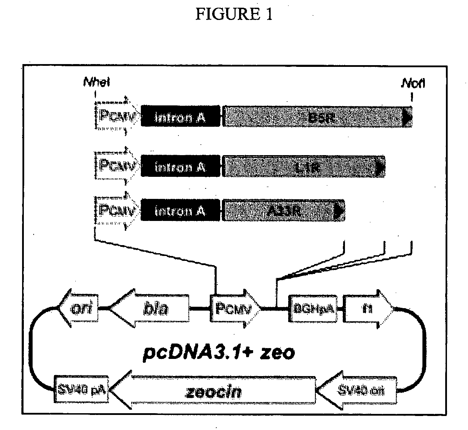

Figure 1 is a plasmid map of pcdna3.1zeo:VACV_B5R, pcdna3.1zeo:VACV_A33R, and pcdna3.1zeo:VACV_LIR.

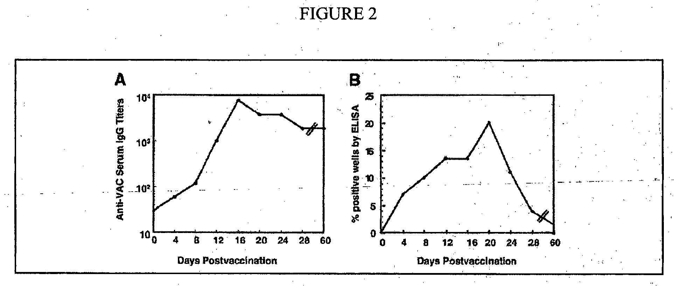

Figure 2A and 2B are graphs of the antibody titre of circulating VACV-specific antibodies and B cells.

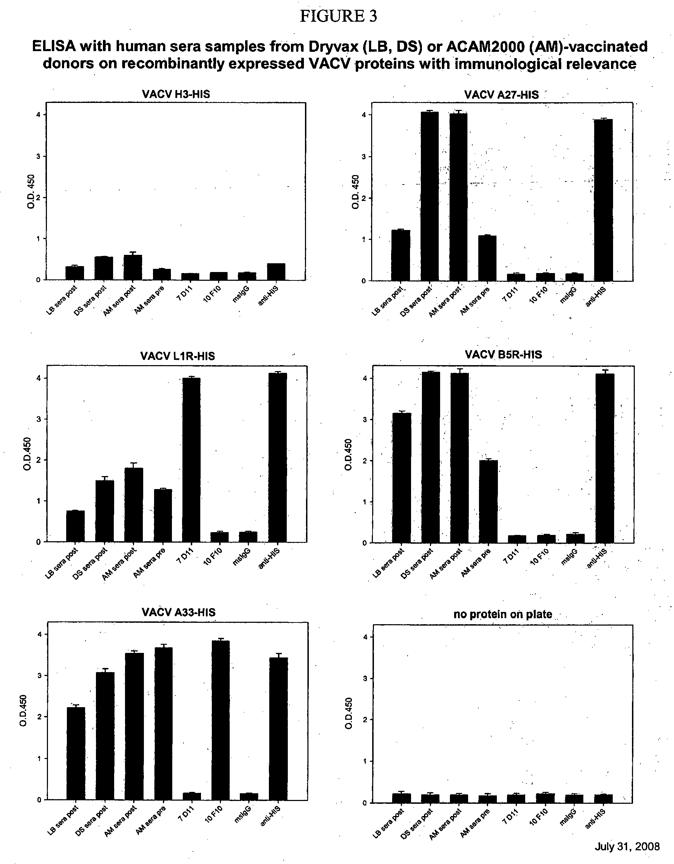

Figure 3 shows the results of ELISA assays of human sera samples from Dryvax (LB,DS) or ACAM2000 (AM) vaccinated donors on recombinantly expressed VACV proteins with immunological relevance.

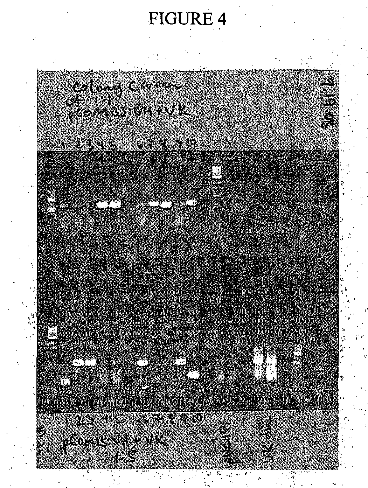

Figure 4 shows the results of a PCR colony analysis of 10 XL-1 blue/ pCOMB:HC+LC κ library clones at vector:insert ratios of 1:1 (top panel) or 1:5 (bottom panel) for presence of a cloned human LC κ cDNA insert.

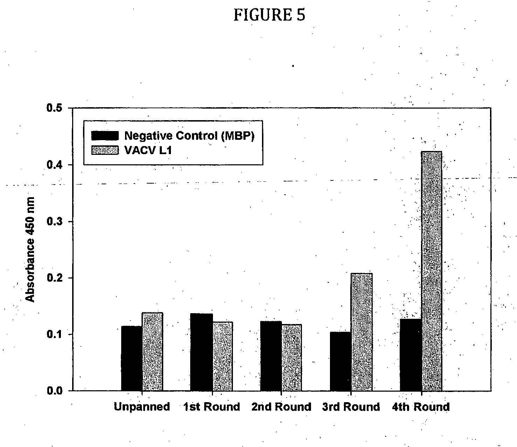

Figure 5 shows results of phage ELISA from different rounds of panning against L1 and MBP.

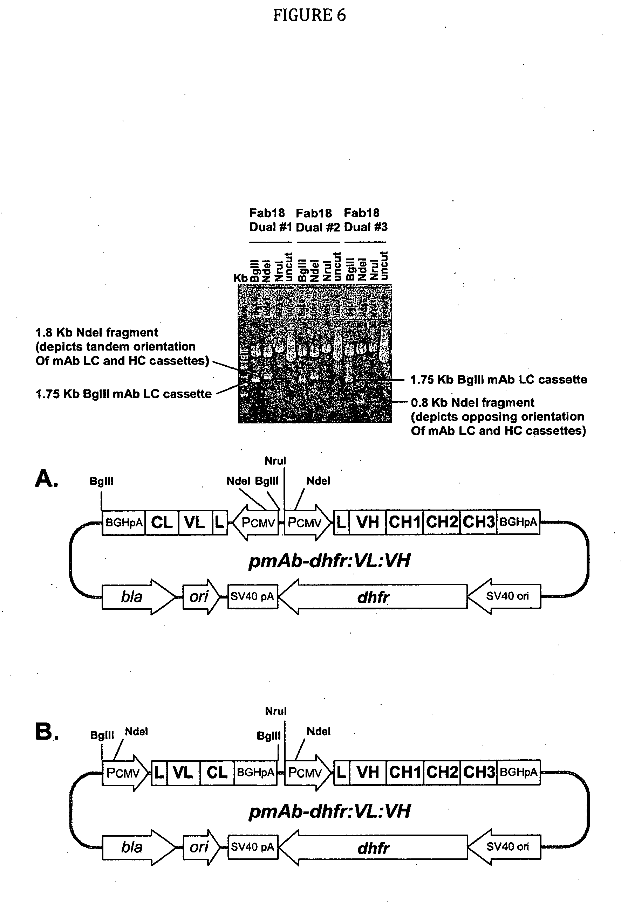

Figure 6 is a photograph of an agarose gel picture and plasmid maps of pcDNAO:dhfr:VL:VH depicting two possible orientations for light and heavy chain cassettes.

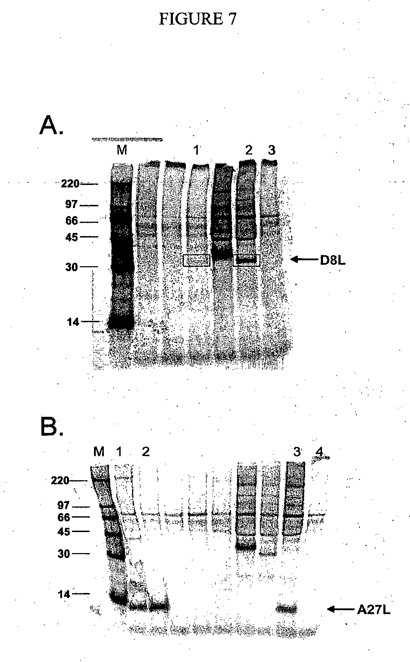

Figure 7 shows the results of an inimunoprecipitation and polyacrylamide gel electrophoresis of radiolabeled VACV proteins with VACV-immune human sera or with mammalian cell-expressed full-length human mAbs.

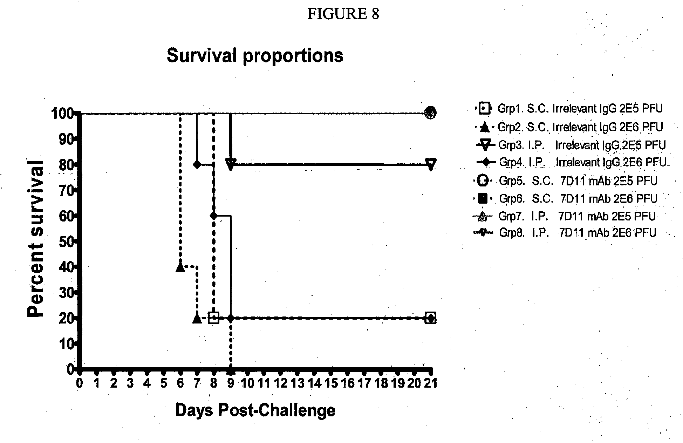

Figure 8 shows the protection of BALB/c mice by monoclonal antibody.

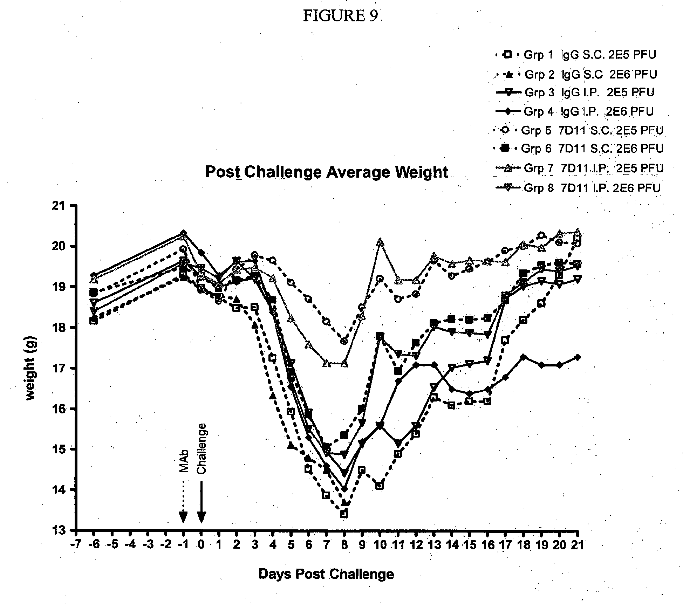

Figure 9 shows weight change after i/n challenge with 2 x 10⁶ or 2 x 10⁵ PFU Vaccinia Virus IHD-J Strain. Average weight of each treatment group (n=5) are shown.
Figure 10 shows the design of a 12-zone microfluidic surface. Each zone may comprise a rectangular gold electrode to permit site specific addressing of functionally-conjugated chitosan mixtures.
Figure 11 shows the design of the microfluidics flow chamber detailing the location of the electrode for electrodepostion of biofunctionalized surface and flow paths.
Figure 12 shows a diagram of conjugated antigen mediated memory B cell separation and soluble ligand activation strategy.
Figure 13 shows FACS data from PBMC thaw (Plot on left is in PBS alone and plot on right is PBS plus PI).
Figure 14 shows the VACV L1 variants with multiple N- and C-terminal tags.
Figure 15 shows agarose gels of (A) PCR product of L1R ectodomain and (B) PCR products of six terminally tagged variants of L1R.
Figure 16 shows an agarose gel of Hind III and Not I restricted mammalian expression plasmid pcdna3.1 +zeo:intA: VACVsL1R+His#1.
Figure 17 shows an agarose gel of Hind III and Not I restricted mammalian expression plasmids containing six L1 terminally tagged variants.
Figure 18 shows an SDS-PAGE gel of transiently expressed purified L1 variants.
Figure 19 shows the results of an ELISA analysis of transiently expressed purified L1 variants.

### DETAILED DESCRIPTION OF THE INVENTION

Several opportunities exist to improve the current discovery and manufacturing approaches for therapeutic mAbs. Our prior results strongly suggest that the use of peripheral blood mononuclear cells (PBMCs) from immunized donors may provide a source of B cell clones that express B cell receptors (BCRs), in the case of memory B cells, or secrete IgG, in the case of antibody secreting cells, that neutralize virus. The current problem areas in the Fab phage display discovery strategies demonstrate issues related to the amplification and manipulation of nucleic acid sequences and the inherent instabilities of the phage display systems. An alternate approach that preserves the advantages of using the human donor B cells, yet avoids the problems associated with gene manipulation is to isolate the specific B cells of interest directly. The circulating pool of antigen-specific memory B cells may also convey the added value of the natural affinity maturation process that occurs during the late adaptive immune response.⁴⁻⁹ These circulating antigen-specific memory B cells may provide antibodies of the highest affinity and potency. In addition, an approach that results in manufacturing cell lines directly derived from the original human antigen- specific memory B cells through the processes of immortalization may provide significant advantages including cell line stability, antibody quality/fidelity, reduced speed to clinic, and lower cost of therapeutic development.¹⁰⁻¹³ Through the use of a biopolymer scaffold to present antigen and controlled microfluidics, B cells of interest may be captured, separated by affinity, activated, and cultured in a single device. As an added benefit, such a platform may be useful in the investigation of the human immune response.

The device for the isolation of immune cells for example antigen-specific memory B cells for the discovery and manufacture of therapeutic monoclonal antibodies.

Having now described the present invention in detail, the same will be more clearly understood by reference to the following examples, which are included herewith for purposes of illustration only and are not intended to be limiting of the invention.

Experiments were designed to demonstrate isolation of VACV L1 -specific memory B cells from the same ACAM2000 vaccinated donor used to generate the Fab phage display library using recombinant L1 antigen. The donor was vaccinated approximately 6 months ago and, based on recent studies, ¹⁴ should currently maintain on the order of 1% VACV-specific circulating memory B cells per total IgG⁺ circulating memory B cells. It has been established that IgG⁺ memory B cells comprise 10-15% of the peripheral B cell compartment.¹⁵ It has also been established that all B cells comprise approximately 10% of circulating PBMCs.¹⁶ Thus, the number of VACV-specific 1gG⁺ circulating memory B cells is on the order of 0.01% of total PBMCs. Assuming 1x10⁶ PBMCs per mL of whole blood, the total number of VACV-specific IgG⁺ circulating memory B cells per mL of whole blood is approximately 100. Of that total number of cells, if we assume that VACV L1 -specific memory B cells make up 1-10%, then we may expect approximately 1-10 cells per mL of whole blood. Therefore, 100 mL of the ACAM2000 vaccinated donor blood may be processed to isolate 100-1000 VACV L1 -specific memory B cells.

### EXAMPLES

### EXAMPLE 1

Recent efforts have illustrated the unique properties of chitosan, a linear b-1.4-linked polysaccharide generated by the partial deacetylation of chitin, and its use in functional biofabrication.¹⁹⁻²³ Two key properties of this biopolymer include its pH-dependent solubility and the reactivity of its amine groups in enzymatic conjugation. The former property permits the spatially directed assembly of chitosan via electrodepositon by exploiting the pH gradient localized at the cathode surface. Studies have demonstrated the assembly of functionally conjugated chitosan in stable thin films onto micropatterned gold electrodes in a microfluidic device.²⁴ The latter property permits the enzymatic assembly of functionally conjugated chitosan. In 2008, Wu, *et. al.* described the conjugation of pentatyrosine-tagged modified protein G to an electrodeposited chitosan biopolymer surface mediated by the enzyme tyrosinase and self-assembly of a fluorescently labeled IgG antibody to that functional surface.²⁵ Further demonstrations of the utility of the biopolymer chitosan include the programmable assembly of a functional metabolic pathway enzyme in a pre-packaged, microfluidic reusable bioMEMS device²⁶ and *in situ* activation and assembly of green fluorescent protein (GFP) onto micropatterned gold electrodes within a multichannel microfluidic device.²⁷ Although chitosan is a preferred polymer, other suitable polymers may be used, for example, polyethylene glycol (PEG), polyacrylamide, etc.

The recruitment of leukocytes such as neutrophils to sites of inflammation is mediated by receptor-ligand interactions. The homing of circulating neutrophils is initiated by the physiological process known as cell rolling. Glycoprotein receptors such as selectins allow for transient adhesive bonds between the leukocyte and the activated endothelium of the blood vessel.²⁸ These bonds have high dissociation rates and are responsive to shear stress.²⁹⁻³⁰ This cell rolling phenomenon has been exploited *in vitro* to permit the separation and two-dimensional nanomechanical control of cells.³¹⁻³² Fractionation of a mixture of cell lines mediated by immobilized E-selectin in a microfluidic device was demonstrated by Chang, *et. al.³³* Separation of stem and progenitor cell populations from other adult bone marrow cells has been accomplished by differential rolling adhesion on L-selectin substrates.³⁴ Studies using IgG coated microspheres in a parallel plate flow chamber were used to correlate increasing critical shear rates for microsphere detachment with increasing ligand surface concentration.³⁵

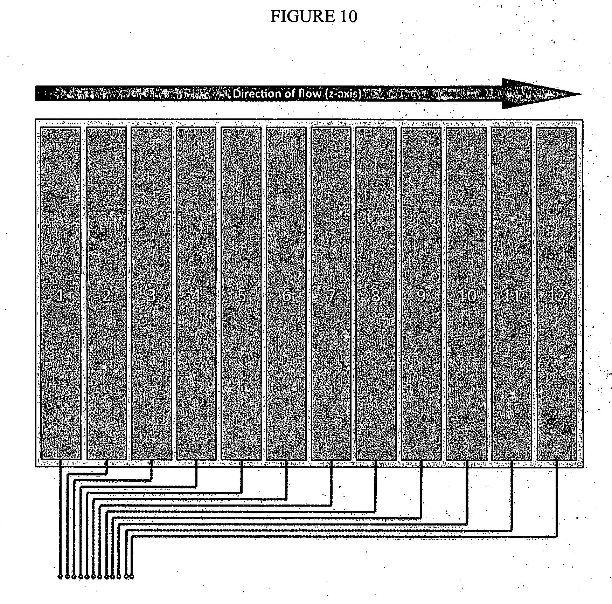

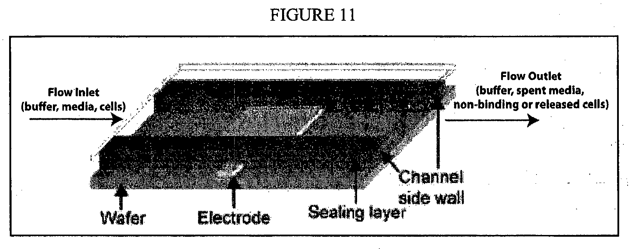

The present invention includes a microfluidic device that will differentially separate antigen-specific B cells based on BCR affinity. As shown in Figure 10, a microfluidic device of the invention may comprise a plurality of zones and each zone may consist of a rectangular gold electrode or electrode array to permit site specific addressing of functionally-conjugated chitosan mixtures. The flow chamber of the device is designed and will be fabricated to include a sealed flow path to permit sterile operation. The electrodes may be integrated into the flow chamber to allow for electrodepostion of functionally conjugated chitosan or other polymer. Flow chamber design is detailed in Figure 11.

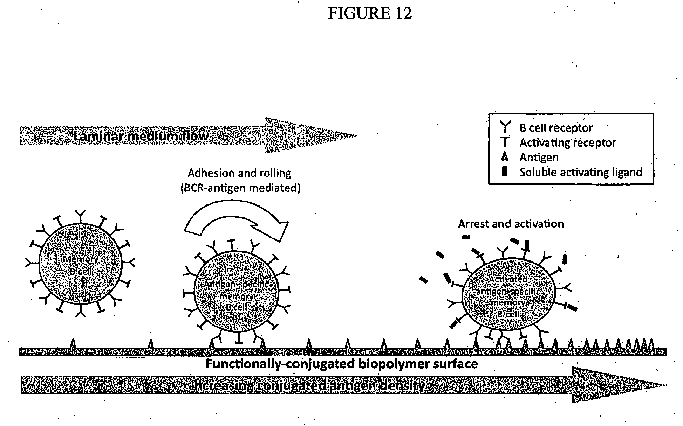

The His-tagged soluble vaccinia virus antigen L1 described above was modified with the addition of a pentatyrosine tag or pentaglutamine to the C-terminus. This antigen may be conjugated to chitosan or other polymer directly or via a tether as described herein. A microfluidic device may be constructed to permit the flow of solutions, culture medium, and B cells across a functional surface. This surface may be electrodeposited with a gradient of VACV L1 antigen or other antigen conjugated chitosan or other polynmer. The antigen conjugated chitosan or other polymer may be electrodeposited in a step gradient along the z-axis of the device in a thin film. The functional surface is designed to accommodate a spatially distributed set of 100-1000 antigen-specific memory B cells. The cells may be introduced to the device and biopolymer surface by laminar flow of cell culture medium. Memory B cells that display BCRs specific for epitopes on the chitosan-anchored L1 interact with the antigen and roll along the surface in the fashion of leukocyte rolling. Since the surface concentration of L1 antigen along the z-axis is a gradient, the interactions are additive, cell rolling slows, and a set of antigen- specific B cells will be arrested on the functionalized surface (see Figure 12). As an additional benefit, the affinity of the BCRs and the resulting secreted mAb can be screened by this differential adhesion mediated separation. Determination of antibody affinity as a function of migration distance can be assayed by surface plasmon resonance measurements (i.e., Biacore analysis) of secreted mAb. An infusion/withdrawal syringe pump (Kent Scientific Corp.) may be used to control flow rate of medium into the flow chamber thereby accurately controlling shear stress across the antigen-conjugated chitosan surface. To prepare concentration surface gradients of conjugated chitosan or other polymer, two or more syringe pumps can be connected to a common inlet tube to the flow chamber. In some embodiments, the electrodeposition surface may be composed of multiple separate electrodes stacked across the flow surface, from the entrance to the exit (see Figure 10). The number of electrodes will dictate the continuity of the gradient (e.g., the more electrodes, the more continuous the gradient). The syringe pumps control the concentrations of the antigen at each zone. This process can be automated. Initial studies may be performed using microspheres with different anti-L1 mAbs conjugated to the surfaces.

Confirmation of gradient electrodeposition can be accomplished by flowing labeled anti-L1 antibodies over the L1 conjugated biopolymer surface. Initial success criterion is the demonstration of differential migration of a mixture of anti-L1 IgG conjugated microspheres. This indicates that fluid flow induced shear forces allow BCR-antigen bonds to associate and dissociate and cause B cell rolling along the antigen-conjugated biopolymer surface. These microsphere experiments set the initial shear stress range and L1 concentration gradients for immune cell isolation. If the microspheres do not migrate, the addition of a competing antigen-specific mAb or Fab to the mobile phase may cause rolling to occur. Another alternative approach is to begin with static capture of the microspheres to the antigen conjugated surface. Then, begin a gradient of competing antigen-specific mAb or Fab in the mobile phase to induce the detachment of mAb conjugated microspheres with higher off rates. At the end of the gradient, only the microspheres with the lowest off rates and highest affinities will remain. The alternative approaches for the microsphere experiments are translated to immune cell (e.g., B cell) experiments using polyclonal competing antigen-specific mAb or Fab. Yet another alternative approach is to conjugate a selectin to chitosan, add it to the gradient, and reduce the surface concentration of L1 antigen or put the antigen downstream to set up a "capture zone." This begins the rolling process non-specifically but arrests the antigen-specific cells.³⁶

### EXAMPLE 2

Cells arrested on the antigen-conjugated chitosan or other polymer surface may be activated to induce differentiation to antibody secreting cells (ASCs) and clonal expansion. Recent and historic studies have identified soluble immune cell (e.g., B cell) activators.³⁷⁻⁴⁵ B cell-activating factor of the TNF family (BAFF) that cooperates with signals emanating from BCR have been shown to stimulate B cell survival, class switch DNA recombination, and antibody production.⁴⁶ In cooperation with IL-10, microbial derived products including CpG-containing oligodeoxynucleotides (ODNs) and bacterial DNA can activate human B cells by turning on the TLR9 pathway.⁴⁷⁻⁴⁹ Recombinant CD40L, IL-4, IL-5, IL-6, IL-15, IL-21 or other supplements may be added to increase activation, differentiation, proliferation, and selection of mAb isotype (*i.e..* IgG). Once activation and expansion is underway, B cells will form colonies of ASCs. Other suitable activators known to those skilled in the art may be used to activate immune cells of other lineages than B cell.

Prior to experiments in the flow chamber, activation medium formulations may be tested on magnetic bead separated antigen-specific memory B cells. Isolation may be performed and cells seeded in 96-well cell culture plates. A commercially available human immune cell medium such as Lymphocyte Growth Medium-3 (Lonza) may be used to culture the B cells. Matrix experiments can be performed with combinations of the activation supplements cited above and include the addition of the soluble recombinant antigen L1. Initial success criterion includes the activation and expansion of memory B cells as indicated by IgG secretion and cell colony formation, respectively. Once achieved, the activation process may be translated to the flow chamber. Cell culturing considerations such as oxygen mass transport may dictate the modification of the flow chamber to permit gas exchange during B cell activation and expansion.

### EXAMPLE 3

The flow chamber used to separate, activate, and colonize B cells is designed to permit visualization of colonies microscopically and facilitate colony transfer using a micromanipulator in an aseptic environment. These colonies may be transferred to 96-well plates for additional expansion and screening of supernatants for antigen-specific mAbs via ELISA methods. mAbs in these supernatants can be further characterized for antigen binding/affinity via surface plasmon resonance (Biacore) to determine correlation of antibody affinity as a function of B cell migration distance.

Flow chamber design may be functionally tested for sterility, colony visualization, and transfer. These tests may be performed with antibiotic-free medium and sterile hold steps prior to cell culture experiments. If activation and cultivation of certain antigen-specific B cells in the microfluidic device is not possible, detachment and transfer of cells to plates after capture may be necessary. This alternative approach will dictate additional limiting dilution cloning steps to isolate individual mAbs.

### EXAMPLE 4

Recombinant soluble VACV L1 was biotinylated using the EZ-Link MicroNHS-PEO₄-Biotinylation Kit (Pierce). Biotinylated L1 was analyzed for reactivity to L1-specific IgG (mAb 7D11 and mAb 10F5) by ELISA. Compared with non-biotinylated L1, the biotinylated reagent exhibited an average 34% and 28% reduction in OD signal for mAb 7D11 and mAb 10F5, respectively. The biotinylated L1 reagent was then analyzed to determine the average number of biotin molecules conjugated to each L1 molecule. This was performed with the Pierce Biotin Quantitation Kit (Cat. No. 28005) which uses HABA (4'-hydroxyazobenzene-2-carboxilic acid) and avidin in a competitive reaction. The result of the experiment showed that an average of 2.2 molecules of biotin are conjugated to each L1 molecule.

In a further characterization experiment, simultaneous reactivity to both anti-L1 and anti-biotin antibodies was demonstrated. Briefly, an L1 capture ELISA was designed by coating a 96-well plate with anti-L1 mAb (7D11). After incubating overnight, the plate was washed and blocked. Blocking buffer was removed and serially diluted samples of biotinylated and non-biotinylated L1 were added to wells. A negative control of blocking buffer with no protein was used to determine background signal. The plate was incubated and then washed. A secondary reagent of anti-biotin mAb was added to all wells. This reagent was sourced from Miltenyi Biotec (Cat. No. 130-090-857). After incubation and washing, rabbit anti-mouse IgG1 was added. After incubation and washing, HRP-conjugated goat anti-rabbit IgG reagents were added. After a final incubation and washing, the plate was developed with an ABTS substrate solution and read on a plate reader at 405 nm. Signals (optical density, OD) from the biotinylated L1 wells were approximately 10 fold higher than both the non-biotinylated wells and the negative (background) wells. Signals from non-biotinylated and negative wells were comparable. These results indicate that the biotinylated L1 reagent can simultaneously bind both an anti-L1 mAb and an anti-biotin mAb and should be suitable for B cell capture experiments.

### EXAMPLE 5

A healthy human volunteer (Donor BB) was bled for serum and circulating lymphocyte isolation prior to ACAM 2000 smallpox vaccination on 07JUL09. Approximately 20 mL of blood was drawn into Vacutainer clot activator collection tubes (BD, Ref. 3678230). The contents were centrifuged at 1000Xg for 20 minutes. Serum fraction was aspirated, aliquoted at 500 µL into cryotubes, and stored in a liquid nitrogen vapor phase dewar. Approximately 75 mL of blood was drawn into Vacutainer collection tubes with sodium heparin (BD, Ref. 367874). Fifteen (15) mL of Histopaque-1077 was added to each of 5 50-mL conical tubes. Fifteen (15) mL of whole blood was carefully overlaid onto each of the Histopaque-1077 aliquots. The tubes were centrifuged at 400Xg for 30 minutes and the peripheral blood mononuclear cell (PBMC) layers were aspirated. The PBMCs were mixed with PBS at a PBS:PBMC ratio of 5:1. The solutions were centrifuged, supernatant was decanted, and PBMCs were resuspended in a total volume of 10 mL of freezing medium (90%FBS/10% DMSO). Cells were aliquoted at 500 µL into cryotubes and stored in a liquid nitrogen vapor phase dewar. Cell count of 1.136X10⁷ viable cells per mL in a total volume of ~11.5 mL indicated a PBMC recovery of approximately 20% based on a recent CBC analysis of Donor BB.

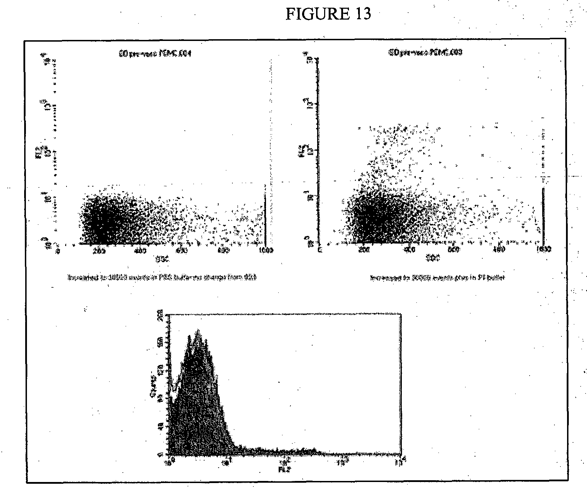

To determine initial suitability and viability of PBMCs for flow cytometer analyses, one aliquot of PBMCs were rapidly thawed and cells were diluted 1:10 into either PBS or PBS with 0.5 µg/mLpropidium iodide (PI). These samples were run on a BD FACSVantage instrument. As shown in Figure 13, cells stained with PI showed nearly 100% viability. PBMCs will be isolated as above at the 21-day post-vaccination time point from Donor BB. In addition, B cell-specific fluorescently labeled Abs will be used to analyze and enrich specific B cell compartments.

### EXAMPLE 6

Several variants of the VACV L1R antigen gene were designed and generated. Specifically, glutamine- and tyrosine-tagged versions of the L1R ectodomain were engineered and PCR assembled. Each of these variants includes a 3' (C-terminal) 6-histidine tag for ease of purification. The Tyr and Gln tags are used in studies to conjugate the proteins to chitosan directly and indirectly via a peptide tether, respectively. Chitosan or other biopolymer will be thin-layer electrodeposited onto a microfluidics chip. Once the VACV L1 antigen is conjugated to the chitosan or polymer surface, capture of antigen-specific B cells will be investigated with both model cell lines and human donor circulating lymphocyte pools. Genes for each of these L1 variants were assembled and cloned into a mammalian expression plasmid. Small-scale transient transfection and affinity purification were used to generate quantities of each protein for analysis by ELISA and conjugation studies.

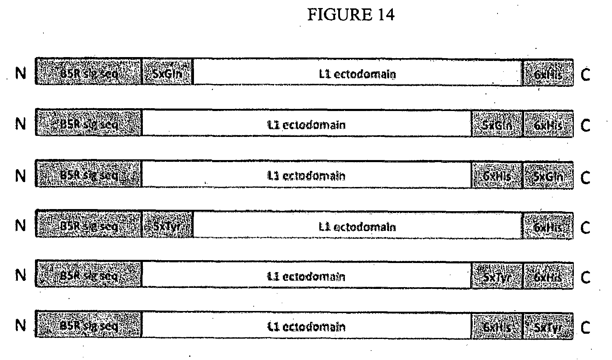

Primers were designed and synthesized and used to generate six PCR products with the various terminal tags (see Figure 14). This PCR assembly was performed in two steps. First, the L1 ectodomain was PCR amplified from a previous construct pcdna3.1+zeo:intA:VACVsL1R+His#1. Second, the gel purified PCR product was used as a master template with the primer sets in Table 5 to generate all six L1 variants.

**Table 5 Primers used for PCR assembly of six terminal tagged VACV L1 variants.**

| | Sequence, 5' to 3' |
|---|---|
| *L1 ectodomain* | |
| L1-F1 | GGTGCCGCGGCAAGCATACAG |
| Ll-Rlrev2 | CTGAACTCCTGTACCAGCAACTTGTTTAGGTGC |
| *L1 with 5' 5xGln tag and 3' 6xHis tag* | |
| L1GlnTag-F2 | CATGAAGCTTCCACCATGAAAACGATTTCCGTTGTT ACGTTGTTATGCGTACTACCTGCTGTTGTTTATTCAA CATGTCAGCAGCAGCAGCAGGGTGCCGCGGCAAGCA TACAG (HindIII) |
| L1HisTag-R2 | CATGGCGGCCGCTCAGTCAGTGATGGTGATGGTGAT GCTGAACTCCTGTACCAGCAAC (NotI) |
| *L1 with 3' 5xGln tag upstream of 3'6xHis tag* | |
| L1-F3 | CATGAAGCTTCCACCATGAAAACGATTTCCGTTGTT ACGTTGTTATGCGTACTACCTGCTGTTGTTTATTCAA CATGTGGTGCCGCGGCAAGCATACAG (HindIII) |
| L1GlnTagHisTag-R3 | CATGGCGGCCGCTCAGTCAGTGATGGTGATGGTGAT GCTGCTGCTGCTGCTGCTGAACTCCTGTACCAGCAAC (NotI) |
| *L1 with 3'5xGln tag downstream of 3' 6xHis tag* | |
| L1-F3 | As above |
| L1HisTagGlnTag-R4 | CATGGCGGCCGCTCAGTCACTGCTGCTGCTGCTGGTG ATGGTGATGGTGATGCTGAACTCCTGTACCAGCAAC (NotI) |
| *L1 with 5'5xTyr tag and 3'6xHis tag* | |
| L1TyrTag-F5 | CATGAAGCTTCCACCATGAAAACGATTTCCGTTGTT ACGTTGTTATGCGTACTACCTGCTGTTGTTTATTCAA CATGTTACTATTACTATTACGGTGCCGCGGCAAGCATA CAG (HindIII) |
| L1HisTag-R2 | As above |
| *L1 with 3'5xTyr tag upstream of 3'6xHis tag* | |
| L1-F3 | As above |
| L1TyrTagHisTag-R6 | CATGGCGGCCGCTCAGTCAGTGATGGTGATGGTGAT GGTAATAGTAATAGTACTGAACTCCTGTACCAGCAAC (NotI) |
| *L1 with 3'5xTyr tag downstream of 3' 6xHis tag* | |
| L1-F3 | As above |
| L1HisTagTyrTag-R7 | CATGGCGGCCGCTCAGTCAGTAATAGTAATAGTAGTG ATGGTGATGGTGATGCTGAACTCCTGTACCAGCAAC (NotI) |

Restriction sites are underlined and listed in braces at end of primer. Gln and Tyr tags are italicized. His tag is bold and italicized.

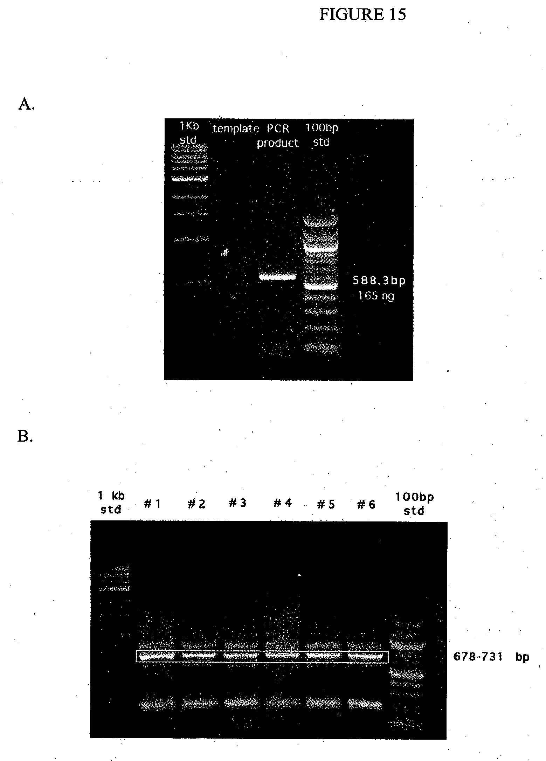

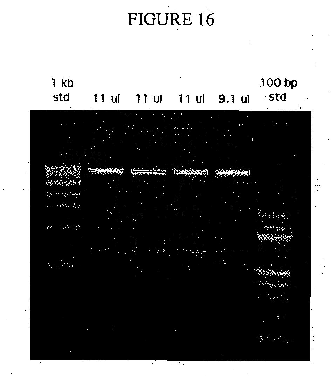

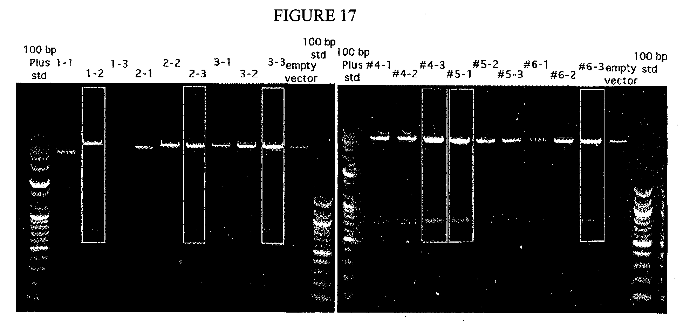

Figure 15 shows the successful two-step PCR assembly. The PCR product bands indicated by the box in Figure 15B were excised and gel purified. The original mammalian expression plasmid pcdna3.1+zeo:intA:VACVsL1R+His#1 was restricted with Hind III and Not I and gel purified at large scale to remove the unmodified L1 ectodomain and provide an acceptor vector for each of the six variants (see Figure 16). The gel purified L1 variants were likewise restricted with Hind III and Not I and then ligated into the acceptor vector. TOP 10 competent E. coli (Invitrogen) were transformed with the plasmids and plated on Carb100 LB agar (100 µg/mL carbenicillin). For each of the six variants, 3 colonies were picked and expanded in 3 mL of Carb50 LB broth at 37°C with shaking for 7 hours. One and one half milliliters of culture were harvested and plasmids were purified using a Qiagen QIAprep Spin Miniprep Kit. One half milliliter of each culture was mixed with an equal volume of 50% glycerol solution and frozen down at -80°C. Plasmid preps were analyzed by restriction endonuclease (REN) using Hind III and Not I. The plasmids indicated by the boxes in Figure 17 were DNA sequence confirmed using primers that complement regions in the plasmid flanking the insert. All sequences were 100% accurate.

Each of the six constructs was prepped at the midi scale for transient transfection and expression. Briefly, for each construct, 30 mL of Carb50 LB broth was inoculated from a single colony of transformed E. coli. Cultures were incubated overnight at 37°C with shaking. The next day, cultures were harvested and plasmids were purified using a Qiagen Plasmid Midi Kit. HEK 293T/17 cells were expanded in T-75 flasks in the following growth medium: DMEM-High Glucose (Invitrogen, Cat. No. 11995-040), 10% FBS-Certified (INVG, Cat. No. 16000-069), 2 mM GlutaMAX (INVG, Cat. No. 35050-061), and 1X NEAA (INVG, Cat. No. 11140-050). At passage 11, cells were counted and seeded in 10 cm cell culture dishes at 3X10⁶ viable cells in 15 mL of growth medium. Cells were incubated for two days and were ~90% confluent. On the day of transfection, medium was replaced with 15 mL of fresh growth medium. For each transfection, 24 µg of plasmid DNA was diluted in 1.5 mL of OptiMEM-I SFM medium (INVG, Cat. No. 31985-062) and 60 µL of Lipofectamine 2000 (INVG, Cat. No. 11668-027) was diluted in 1.5 mL of OptiMEM-I. Solutions were gently combined and incubated at room temperature for 20 minutes. Reactions were added dropwise to each 10 cm cell culture dish and transfections were allowed to proceed for 5.5 days. Supernatants were aspirated and clarified by centrifugation and 0.22 µm filtration.

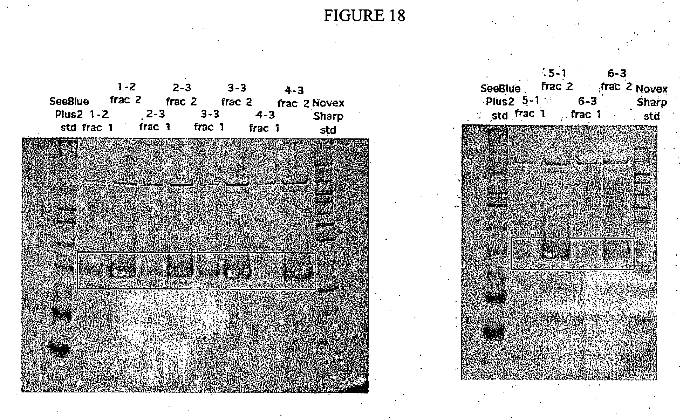

Transiently expressed L1 variants were purified using a HisPur Purification Kit with 3 mL resin bed volume with a spin purification protocol (Pierce, Cat. No. 90092). His-tagged proteins were eluted in two 3 mL fractions using the included elution buffer (50 mM sodium phosphate, 300 mM sodium chloride, 150 mM imidazole; pH 7.4). A total of 26 µL of each fraction was loaded on SDS-PAGE gels in reducing sample buffer (NuPAGE Novex 10% Bis-Tris Gel 1.0 mm, 10 well, INVG, Cat. No. NP0301). Gels were run at 200 V constant for 45 min and stained with coomassie (SimplyBlue SafeStain, INVG, Cat. No. LC6065). Stained SDS-PAGE gels are shown in Figure 18. Major bands indicated in boxes were 25,881-27,234 Da (mean = 26, 601 Da) with a purity of 62-83% by densitometry analysis. Fractions 1 and 2 for each protein were pooled, quantitated by A280, sterile filtered at 0.22 µm, and stored at -20°C.

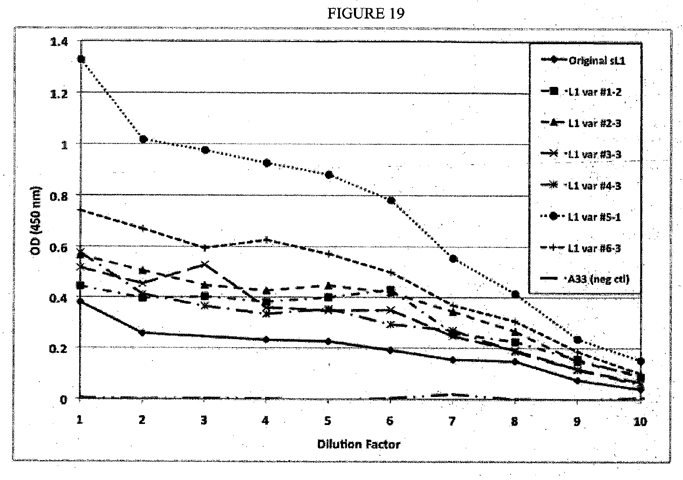

Proteins were analyzed for antibody binding to mAb c7D11 (α-VACV L1 chimeric monoclonal) by ELISA. This analysis will determine if the conformational epitope is conserved indicating proper protein folding (see Su et. al., Virology, 2007, 368(2):331-41 for Structural basis for the binding of the neutralizing antibody, 7D11, to the poxvirus L1 protein). Immediately prior to freeze-down, pooled final purified proteins were used to coat a 96-well ELISA plate at 100 ng per well in PBS. Each protein was coated in the 12 wells of rows A-F. As a positive control, original soluble L1 (His-tagged only) was coated in the 12 wells of row G at 100 ng per well in PBS. As a negative control, soluble VACV A33 subunit protein was coated in the 12 wells of row H at 100 ng per well in PBS. Plates were incubated overnight at 4°C. The plate was washed and blocked with 5% non-fat dry milk (NFDM), 0.1% TWEEN 20, and 0.01% thymerisol in PBS for 45 minutes with shaking at room temperature. Purified c7D11 mAb was diluted in a 1:2 series in blocking buffer beginning with a concentration of 3000 ng/mL for ten dilutions. Blocking buffer was discarded from the ELISA plate and the c7D11 standard curve dilution series was added to each of the rows (100 µL per well) of the plate reserving the last two wells of each row for blocking buffer only (buffer negative control). The plate was incubated for 45 minutes with shaking at room temperature. Samples were discarded and ELISA plate was washed. Secondary antibody (Anti-Human IgG [Fab Specific]-Peroxidase, antibody produced in goat, Sigma, Cat. No. A0293-1ML) was diluted 1:20,000 in blocking buffer and added to each well (100 µL). The plate was incubated for 45 minutes with shaking at room temperature. Secondary was discarded and ELISA plate was washed. Plate was developed with TMB Liquid Substrate System (Sigma, Cat. No. T0440-100ML, 100 µL per well). Reaction was stopped with the addition of 100 µL per well of 0.5 M H2SO4. ELISA plate was read at 450 nm. Figure 19 shows the signal curves produced by each row of coated protein (L1 variants, sLI original, and VACV A33). As seen, all L1 variants demonstrate similar signal response with the mAb c7D11 dilutions and are comparable to or exceed the signal intensities of the original His-tag only sL1. No signal was observed in the A33 or buffer negative control wells. This indicates the preservation of the conformational epitope recognized by mAb c7D11.

### EXAMPLE 7

Individual L1 variants may be selected based on functional and design criteria, conjugated to chitosan or other polymer, and analyzed for functionality on a chip. Chitosan solutions (1% w/v) may be prepared by dissolving chitosan flakes in an aqueous solution of HCl (1% v/v) and adjusting the pH to 5.6 using 1 M NaOH. A gelatin stock solution (5%) may be prepared by dissolving gelatin in 20 mM phosphate buffered saline (PBS, pH 6.5). Both solutions maybe autoclaved at 121 °C for 15 min. Protein assembly maybe performed using a chip prepared by patterning gold electrodes onto a silicon wafer using standard microfabrication. To electrodeposit chitosan, the chip may be partially immersed in the chitosan solution (5 mL, 1% w/v, pH 5.6) and an electrode biased to serve as the cathode at a controlled current density of 5 A/m2 for 1 min (typical voltage was less than 2.5 V). After deposition, the electrode may be disconnected from the power supply and the chip washed with deionized water and soaked in PBS (pH 7.4) for 30 min.

To graft the gelatin tethers onto the electrodeposited chitosan films, the chip may be incubated in 3 mL of 20 mM phosphate buffer (pH 6.5) containing tyrosinase (50 U/mL) and gelatin (0.5-4%). After grafting, the chip may be washed with 10 mL of 20mM phosphate buffer (pH 6.5) containing 0.1% Triton X-100 for three times. Glutamine-tagged L1 variants may be assembled to the tethers by incubating the chip in 3mL of 20mM phosphate buffer (pH 6.5) containing microbial transglutaminase (mTG; 1 U/mL) and the gln-tagged L1 (1.3 µM). Reactions may be performed overnight at room temperature.

To demonstrate specific binding of an anti-L1 monoclonal antibody (mAb), the chip may be immersed in a solution containing mAb c7D11 (α-VACV L1 chimeric monoclonal). The chips may be washed and immersed in an anti-human IgG (Fc-specific) fluorescently labeled secondary antibody. After washing, the fluorescence of the electrode addresses may be observed using a fluorescence microscope (Leica; MZFL III). Fluorescence photomicrographs may be obtained for the chip using a digital camera (Spot 32, Diagnostic Instruments) connected to the fluorescence microscope. The fluorescence profiles of the micrographs may be analyzed using Image J software

### EXAMPLE 8

Capture and expansion of antigen-specific T cells may be accomplished as above with the following modifications: 1) The antigen conjugated to the functional surface in the device may be a peptide with T cell epitopes; 2) The human PBLs may be enriched for T cells (*i.e.,* magnetic bead or flow cytometry methods) prior to introduction into the device for separation; and 3) After T cell separation and arrest on the functionalized surface in the flow chamber, T cells may be activated by the addition of T cell activation medium containing known T cell activation factors. After colonies of clonal T cells form, they may be removed by a micromanipulator, deposited in 96-well cell culture plates, and further expanded.

### References

1. Schmaljohn C, Cui Y, Kerby S, Pennock D., and Spik K. Production and characterization of human monoclonal antibody Fab fragments to vaccinia virus from a phage-display combinatorial library. Virology. 1999; 258:189-200.
2. Barbas CF, Kang AS, Lerner RA, and Benkovic SJ. Assembly of combinatorial antibody libraries on phage surfaces: The gene III site. PNAS. 1991; 88:7978-82.
3. Guttieri MC, Sinha T, Bookwalter C, Liang M, and Schmaljohn CS. Cassette vectors for conversion of Fab fragments into full-length human IgG1 monoclonal antibodies by expression in stably transformed insect cells. Hybrid Hybridomics. 2003; 22:135-45.
4. Franklin A and Blanden RV. Potential inhibition of somatic hypermutation by nucleoside analogues. Mol. Immun. 2007; 44:666-9.
5. Guay HM, Panarey L, Reed AJ, and Caton AJ. Specificity-based negative selection of autoreactive B cells during memory formation. J. Immunol. 2004; 173:5485-94.
6. Low NM, Holliger P, and Winter G. Mimicking somatic hypermutation: Affinity maturation of antibodies displayed on bateriophage using a bacterial mutator strain. J. Mol. Biol. 1996; 260:359-68.
7. Steele EJ. Reflections on the state of play in somatic hypermutation. Mol. Immunol. 2008; 45:2723-6.
8. Steidl A, Ratsch O, Brocks B, Dürr M, and Thomassen-Wolf E. In vitro affinity maturation of human GM-CSF antibodies by targeted CDR-diversification. Mol. Immun. 2008; 46:135-44.
9. Thompson J, Pope T, Tung J-S, Chan C, Hollis G, Mark G, and Johnson KS. Affinity maturation of a high-affinity human monoclonal antibody against the third hypervariable loop of human immuno deficiency virus: Use of phage display to improve affinity and broaden strain reactivity. J. Mol. Biol. 1996; 256:77-88.
10. Kvell K, Nguyen TH, Salmon P, Glauser F, Werner-Favre C, Barnet M, Schneider P, Trono D, and Zubler RH. Transduction of CpG DNA-stimulated primary human B cells with bicistronic lentivectors. Mol. Immun. 2008; 46:135-44.
11. Lanzavecchia A, Bernasconi N, Traggiai E, Ruprecht CR, Corti D, and Sallusto F. Understanding and making use of human memory B cells. Immunol. Rev. 2006; 211:303-9.
12. Li J, Sai T, Berger M, Chao Q, Davidson D, Deshmukh G, Drozdowski B, Ebel W, Harley S, Henry M, Jacob S, Kline B, Lazo E, Rotella F, Routhier E, Rudolph K, Sage J, Simon P, Yao J, Zhou Y, Kavuru M, Bonfield T, Thormassen MJ, Sass PM, Nicolaides NC, and Grasso L. Human antibodies for immunotherapy development generated via a human B cell hybridoma technology. PNAS. 2006; 103:3557-62.
13. Weisner M, Zentz C, Mayr C, Wimmer R, Hammerschmidt W, Zeidler R, and Moosmann A. Conditional immortalization of human B cells by CD40 ligation. PLoS ONE 2008; 1:1-13.
14. Crotty S, Feigner P, Davies H, Glidewell J, Villarreal L, and Ahmed R. Cutting edge: Long-term B cell memory in humans after smallpox vaccination. J. Imm. 2003; 171:4969-73.
15. Klein U, Rajewsky K, and Kuppers R. Human immunoglobulin (Ig)M+IgD+ peripheral blood B cells expressing the CD27 cell surface antigen carry somatically mutated variable region genes: CD27 as a general marker for somatically mutated (memory) B cells. J. Exp. Med. 1998; 188:1679-89.
16. Marti GE, Magruder L, Patrick K, Vail M, Schuette W, Keller R, Muirhead K, Horan P, and Gralnick HR. Normal human blood density gradient lymphocyte subset analysis: An Interlaboratory flow cytometric comparison of 85 normal adults. Amer. J. Hema. 1985; 20:41-52.
17. Babcook JS, Leslie KB, Olsen OA, Salmon RA, and Schrader JW. A novel strategy for generating monoclonal antibodies from single, isolated lymphocytes producing antibodies of defined specificities. PNAS. 1996; 93:7843-8.
18. Guan Y, Sajadi MM, Kamin-Lewis R, Fouts TR, Dimitrov A, Zhang Z, Redfield RR, DeVico AL, Gallo RC, and Lewis GK. Discordant memory B cell and circulating anti-Env antibody responses in HIV-1 infection. PNAS. 2009; epub ahead of print.
19. Yi H, Wu L-Q, Bentley WE, Ghodssi R, RubloffGW, Culver JN, and Payne GF. Biofabrication with chitosan. Macromolecules. 2005; 6:2881-94.
20. Payne GF and Raghavan SR. Chitosan: a soft interconnect for hierarchical assembly of nanoscale components. Soft Matter. 2007; 3:521-7.
21. Lewandowski AT, Bentley WE, Yi H, Rubloff GW, Payne GF, and Ghodssi R. Towards area-based in vitro metabolic engineering: Assembly of Pfs enzyme onto patterned microfabricated chips. Biotech. Prog. 2008; 24:1042-51.
22. Shi X-W, Yang X, Gaskell KJ, Liu Y, Kobatake E, Bentley WE, and Payne GF. Reagentless protein assembly triggered by localized electrical signals. Adv. Mat. 2009; 21:984-8.
23. Yang X, Shi X-W, Liu Y, Bentley WE, and Payne GF. Orthogonal enzymatic reactions for the assembly of proteins at electrode addresses. Langmuir. 2009; 25:338-44.
24. Park JJ, Luo X, Yi H, Valentine TM, Payne GF, Bentley WE, Ghodssi R, and Rubloff GW. Chitosan-mediated in situ biomolecule assembly in completely packaged microfluidic devices. Lab Chip. 2006; 6:1315-21.
25. Wu H-C, Shi X-W, Tsao C-Y, Lewandowski AT, Fernandes R, Hung C-W, DeShong P, Kobatake E, Valdes JJ, Payne GF, and Bentley WE. Biofabrication of antibodies and antigens via IgG-binding domain engineered with activatable pentatyrosine pro-tag. Biotech. Bioeng. 2009; epub ahead of print.
26. Luo X, Lewandowski AT, Yi H, Payne GF, Ghodssi R, Bentley WE, and Rubloff GW. Programmable assembly of a metabolic pathway enzyme in a pre-packaged reusable bioMEMS device. Lab Chip. 2008; 8:420-30.
27. Lewandowski AT, Yi H, Luo X, Payne GF, Ghodssi R, Rubloff GF, and Bentley WE. Protein assembly onto patterned microfabricated devices through enzymatic activation of fusion pro-tag. Biotech. Bioeng. 2008; 99:499-507.
28. Janeway CA, Travers P, Walport M, and Shlomchik MJ. Immunobiology: The immune system in health and disease, 6th Ed. New York: Garland Science, 2005.
29. Dong C and Lei XX. Biomechanics of cell rolling: Shear fow, cell-surface adhesion, and cell deformability. J. Biomech. 2000; 33:35-43.
30. Fritz J, Katopodis AG, Kolbinger F, and Anselmetti D. Force-mediated kinetics of single P-selectin/ligand complexes observed by atomic force microscopy. PNAS. 1998; 95:12283-8.
31. Charles N, Liesveld JL, and King MR. Investigating the feasibility of stem cell enrichment mediated by immobilized selectins. Biotech. Prog. 2007; 23:1463-72.
32. Karnik R, Hong S, Zhang H, Mei Y, Anderson DG, Karp JM, and Langer R. Nanomechanical control of cell rolling in two dimensions through surface patterning of receptors. Nano Lett. 2008; 8:1153-8.
33. Chang WC, Lee LP, and Liepmann D. Biomimetic technique for adhesion-based collection and separation of cells in a microfluidic channel. Lab Chip. 2005; 5:64-73.
34. Greenberg AW and Hammer DA. Cell separation mediated by differential rolling adhesion. Biotech. Bioeng. 2001; 73:111-24.
35. Gao B and Jin G. Study of interaction force between antigen and antibody using flow chamber method. Proc. of IEEE EMB 27th Ann. Conf. 2005; 7584-7.
36. Hong S, Lee D, Zhang H, Zhang JQ, Resvick JN, Khademhosseini A, King MR; Langer R, and Karp JM. Covalent immobilization of P-selectin enhances cell rolling. Langmuir. 2007; 23:12261-8.
37. Bagley KC, Abdelwahab SF, Tuskan RG, and Lewis GK. Cholera toxin indirectly activates human monocyte-derived dendritic cells in vitro through the production of soluble factors, including prostaglandin E2 and nitric oxide. Clinical and Vaccine Immunology 2006; 13:106-15.
38. Bagley KC, Abdelwahab SF, Tuskan RG, and Lewis GK. Pasteurella multocida toxin activates human monocyte-derived and murine bone marrow-derived dendritic cells in vitro but suppresses antibody production in vivo. Infection and Immunity 2005; 73:413-21.
39. Bagley KC, Abdelwahab SF, Tuskan RG, and Lewis GK. Calcium signaling through phospholipase C activates dendritic cells to mature and is necessary for the activation and maturation of dendritic cells induced by diverse agonists. Clinical and Diagnostic Laboratory Immunology 2004; 11:77-82.
40. Bagley KC, Abdelwahab SF, Tuskan RG, Fouts TR, and Lewis GK. Pertussis toxin and the adenylate cyclase toxin from Bordetella pertussis activate human monocyte-derived dendritic cells and dominantly inhibit cytokine production through a cAMP-dependent pathway. J. Leukocyte Biol. 2002; 72:962-9.
41. Brunswick M, June CH, Finkelman FD, Dintzis HM, Inman JK and Mond JJ. Surface immunoglobulin-mediated B-cell activation in the absence of detectable elevations in intracellular ionized calcium: A model for T-cell independence B-cell activation. PNAS 1989; 86:6724-8.
42. Hebeis BJ, Klenovsek K, Rohwer P, Ritter U, Schneider A, Mach M, and Winkler TH. Activation of virus-specific memory B cells in the absence of T cell help. J. Exp. Med. 2004; 199:593-602.
43. Klaus SJ, Pinchuk LM, Ochs HD, Law C-L, Fanslow WC, Armitage RJ, and Clark EA. Costimulation through CD28 enhances T cell-dependent B cell activation via CD40-CD40L interaction. J. Immun. 1994; 152:5643-52.
44. Li W. Synergistic antibody induction by antigen-CD40 ligand fusion protein as improved immunogen. Immun. 2005; 115:215-22.
45. Li X, Vanitha DJ, Joo HM, He Y, Rouse BT, and Sangster MY. A strategy for selective, CD4+ T cell-independent activation of virus-specific memory B cells for limiting dilution analysis. J. Immun. Meth. 2006; 313:110-8.
46. Schneider P, MacKay F, Steiner V, Hofmann K, Bodmer JL, Holler N, Ambrose C, Lawton P, Bixler S, and Acha-Orbea S. BAFF, a novel ligand of the tumor necrosis factor family, stimulates B cell growth. J. Exp. Med. 1999. 189:1747.
47. He B, Qiao X, and Cerutti A. CpG DNA induces IgG class switch DNA recombination by activating human B cells through an innate pathway that requires TLR9 and cooperates with IL-10. J. Immun. 2004; 173:4479-91.
48. Huggins J, Pellegrin T, Felgar RE, Wei C, Brown M, Zheng B, Milner ECB, Bernstein SH, Sanz I, and Zand MS. CpG DNA activation and plasma-cell differentiation of CD27-naïve humand B cells. Blood. 2007; 109:1611-9
49. Richard K, Pierce SK, and Song W. The agonists of TLR4 and 9 are sufficient to activate memory B cells to differentiate into plasma cells in vitro but not in vivo. J. Immunol. 2008; 181:1746-52.

While the foregoing invention has been described in some detail for purposes of clarity and understanding, it will be appreciated by one skilled in the art from a reading of this disclosure that various changes in form and detail can be made without departing from the true scope of the invention and appended claims.

## Claims

1. A device for the separation of immune cells by interactions of receptors of said immune cells with ligands wherein the ligands are immobilized in a concentration gradient on a functionalized surface of the device and the device is adapted to flow the immune cells across the functionalized surface, wherein the concentration gradient of the ligands increases along a direction of flow of the immune cells across the functionalized surface.

2. The device of claim 1, wherein said device is a flow cell or microfluidics device.

3. The device of claim 1, wherein said immune cells are B cells.

4. The device of claim 1, wherein said immune cell receptors are B cell receptors.

5. The device of claim 1, wherein said ligands are antigens with B cell epitopes.

6. The device of claim 1, wherein said immune cells are T cells.

7. The device of claim 1, wherein said immune cell receptors are T cell receptors.

8. The device of claim 1, wherein said ligands are antigens with T cell epitopes.

9. The device of claim 1, wherein said functionalized surface is a polymer conjugated to said ligands.

10. A method for isolating immune cells, comprising: contacting a solution comprising the immune cells with a device according to claim 1.

11. A method according to claim 10, wherein the immune cells are arrested on said functionalized surface and are caused to proliferate by induction of activation.

12. A method according to claim 11, wherein viable activated proliferating immune cell colonies are recovered from the device.

13. A method according to claim 12, wherein colonies are recovered by releasing the ligand from the polymer.

14. A method according to claim 12, wherein the colonies are mechanically removed.

## Patentansprüche

1. Vorrichtung zum Trennen von Immunzellen auf Basis von Wechselwirkungen von Rezeptoren der Immunzellen mit Liganden, wobei die Liganden in einem Konzentrationsgradienten auf einer funktionalisierten Oberfläche der Vorrichtung immobilisiert sind und die Vorrichtung dazu ausgelegt ist, die Immunzellen über die funktionalisierte Oberfläche zu strömen, wobei sich der Konzentrationsgradient der Liganden entlang einer Strömungsrichtung der Immunzellen über die funktionalisierte Oberfläche erhöht.

2. Vorrichtung nach Anspruch 1, wobei die Vorrichtung eine Durchflusszelle oder eine mikrofluidische Vorrichtung ist.

3. Vorrichtung nach Anspruch 1, wobei die Immunzellen B-Zellen sind.

4. Vorrichtung nach Anspruch 1, wobei die Immunzellrezeptoren B-Zell-Rezeptoren sind.

5. Vorrichtung nach Anspruch 1, wobei die Liganden Antigene sind, die B-Zell-Epitope aufweisen.

6. Vorrichtung nach Anspruch 1, wobei die Immunzellen T-Zellen sind.

7. Vorrichtung nach Anspruch 1, wobei die Immunzellrezeptoren T-Zell-Rezeptoren sind.

8. Vorrichtung nach Anspruch 1, wobei die Liganden Antigene sind, die T-Zell-Epitope aufweisen.

9. Vorrichtung nach Anspruch 1, wobei die funktionalisierte Oberfläche ein mit den Liganden verbundenes Polymer ist.

10. Verfahren zur Isolierung von Immunzellen, umfassend das Inkontaktbringen einer die Immunzellen umfassenden Lösung mit einer Vorrichtung nach Anspruch 1.

11. Verfahren nach Anspruch 10, wobei die Immunzellen auf der funktionalisierten Oberfläche festgehalten und durch Induktion der Aktivierung zur Proliferation gebracht werden.

12. Verfahren nach Anspruch 11, wobei Kolonien lebensfähiger, aktivierter, proliferierender Immunzellen aus der Vorrichtung gewonnen werden.

13. Verfahren nach Anspruch 12, wobei die Kolonien durch Freisetzen des Liganden aus dem Polymer gewonnen werden.

14. Verfahren nach Anspruch 12, wobei die Kolonien mechanisch entfernt werden.

## Revendications

1. Dispositif permettant la séparation de cellules immunitaires par des interactions des récepteurs desdites cellules immunitaires avec des ligands dans lequel les ligands sont immobilisés selon un gradient de concentration sur une surface fonctionnalisée du dispositif et le dispositif est conçu pour permettre l'écoulement des cellules immunitaires sur la surface fonctionnalisée, le gradient de concentration des ligands allant croissant dans le sens d'écoulement des cellules immunitaires sur la surface fonctionnalisée.

2. Dispositif selon la revendication 1, dans lequel ledit dispositif est un dispositif microfluidique ou à cellule d'écoulement.

3. Dispositif selon la revendication 1, dans lequel lesdites cellules immunitaires sont des lymphocytes B.

4. Dispositif selon la revendication 1, dans lequel lesdits récepteurs de cellules immunitaires sont des récepteurs de lymphocytes B.

5. Dispositif selon la revendication 1, dans lequel lesdits ligands sont des antigènes comportant des épitopes de lymphocytes B.

6. Dispositif selon la revendication 1, dans lequel lesdites cellules immunitaires sont des lymphocytes T.

7. Dispositif selon la revendication 1, dans lequel lesdits récepteurs de cellules immunitaires sont des récepteurs de lymphocytes T.

8. Dispositif selon la revendication 1, dans lequel lesdits ligands sont des antigènes comportant des épitopes de lymphocytes T.

9. Dispositif selon la revendication 1, dans lequel ladite surface fonctionnalisée est un polymère conjugué auxdits ligands.

10. Procédé d'isolement de cellules immunitaires, comprenant : la mise en contact d'une solution comprenant les cellules immunitaires avec un dispositif selon la revendication 1.

11. Procédé selon la revendication 10, dans lequel les cellules immunitaires sont arrêtées sur ladite surface fonctionnalisée et sont amenées à proliférer par induction de l'activation.

12. Procédé selon la revendication 11, dans lequel les colonies de cellules immunitaires activées viables, en cours de prolifération sont récupérées à partir du dispositif.

13. Procédé selon la revendication 12, dans lequel les colonies sont récupérées par libération du ligand du polymère.

14. Procédé selon la revendication 12, dans lequel les colonies sont retirées mécaniquement.
